# EUROPEAN PATENT APPLICATION

(11) **EP 4 227 422 A1**
(43) Date of publication of application: **16.08.2023**
(21) Application number: 22162040.4
(22) Date of filing: 15.03.2022
(51) Int. Cl.: C12Q 1/6806, C12Q 1/6804, C12Q 1/70

(54) **COMPOSITION FOR POC PCR**

(30) Priority: 15.02.2022 EP 22156799
(71) Applicant: Procomcure Biotech GmbH, 5303 Thalgau (AT)
(72) Inventor: ÖNDER, Kamil, 5301 Eugendorf (AT)
(74) Representative: dompatent von Kreisler Selting Werner - Partnerschaft von Patent- und Rechtsanwälten mbB

(57) **Abstract**

Liquid composition for the preparation of a biological sample for methods for assaying for the presence of SARS-CoV-2 or genetic variations (mutant) of SARS-CoV-2 wildtype in said sample by POC PCR comprising:
a) a chaotropic salt, preferably guanidinium thiocyanate, in a concentration ranging from 1 to 960 mM and
b) optionally one or more RNAse inhibitor.

## Description

The present invention relates to a liquid composition for the preparation of a biological sample for methods for assaying for the presence of SARS-CoV-2 or genetic variations (mutant) of SARS-CoV-2 wildtype in said sample by POC PCR as well as the use of said composition for POC PCR and a POC PCR method for the detection of SARS-Cov-2 or mutants thereof.

Severe acute respiratory syndrome coronavirus 2 (SARS-CoV-2) is an RNA based virus that causes coronavirus disease 2019 (COVID-19). SARS-CoV-2 with a diameter ranging from about 60 to 140 nm has spike, envelope, membrane and nucleocapsid proteins. Spike protein is the one for allowing the virus to bind to the receptor ACE2 of a host cell and to enter the host cell. A patient infected with SARS-CoV-2 may suffer from severe pneumonia and acute respiratory distress syndrome. Since the first infection case of SARS-CoV-2 reported in December 2019, SARS-CoV-2 has spread across the whole world, which causes severe problems in medical systems, economics and other social issues. Till early October 2020, World Health Organization (WHO) has reported more than 35 million cases of infection and 1 million deaths in the world. In addition to other control measures, e.g. social distance and hand washing, WHO has strongly recommended a large amount of testing not only of symptomatic persons and their contacts but also of asymptomatic contacts. Therefore, large amounts of tests are one of the most important measures to control the outbreak of SARS-CoV-2. To date, polymerase chain reaction (PCR) based tests with high sensitivity and specificity are the gold standard for the diagnostics of SARS-CoV-2 infection. PCR tests include several complex experimental procedures, which costs at least a few hours to get the test results. In a PCR test, most experimental procedures require sophisticated and very expensive instrumentation and should be handled by experienced experts. Otherwise, there is a high chance for a false positive/negative result. It leads to huge efforts for medical staffs in the clinics, especially during the exponential increase in infection cases. In developing and underdeveloped countries, the situation for the diagnostics of SARS-CoV-2 with PCR is even worse due to the lack of equipped labs, instrumentation and experienced experts. Therefore, it is of extreme importance and necessity to develop new methods and/or instrumentation for rapid diagnostics of SARS-CoV-2 with reasonable costs. WHO highly encourages researches on the performance of rapid diagnostics approaches and potential diagnostic utilities.

Point-Of-Care (POC) SARS-CoV-2 tests, which can deliver results in a matter of minutes, are proposed to shift away from traditional clinical diagnostical lab tests that take days to POC-tests that can be performed in the field.

These POC tests are based on a variety of technologies and formats both old and new: conventional antigen-based tests for detecting viral proteins; isothermal nucleic acid amplification and loop-mediated isothermal amplification (LAMP) for the detection of viral genetic material; and newer CRISPR-based diagnostic tests, which detect viral genetic material but in fewer steps and dispense with polymerase chain reaction (PCR) machines. POC tests are easy to use as they can be deployed in high-risk environments, such as homes or educational institutions, and require little or no specialist training. Furthermore, some POC tests will be suitable for home use once given the go-ahead by regulators. In this way, POC tests promise to be, at the very least, a useful adjunct to lab-based PCR diagnostic testing.

SARS-CoV-2 antigen tests are cost-effective as they are based on the same lateral flow immunoassay (LFIA) format used in home pregnancy tests, and could help track and contain the spread of the pandemic due to ease of use in clinics and eventually homes. Although the sensitivity of antigen-based POC tests is about two orders of magnitude lower than that of a PCR test, the repeated rapid testing of the general public, also known as surveillance testing, could do a better job at identifying contagious individuals than reliable but cumbersome PCR tests. Large-scale rollouts of POC antigen tests have yet to be deployed, but if successfully done, will set a precedent for rapid development and deployment of antigen tests should another Covid-19 outbreak or pandemic occur. Low-income countries cannot afford population-wide testing but are interested in POC antigen testing as a core diagnostic strategy due to its cost-effectiveness.

Covid-19 antibody tests serve as a cautionary tale, as at the beginning of the pandemic back in March and April 2020, there was great excitement for Covid-19 antibody tests to provide "immunity" testing for those who had already had Covid-19 and now perhaps were immune to the disease. However, time has shown this furor to be misplaced as the initial uptake of Covid-19 antibody tests has still yet to find a strong foothold and function in the Covid-19 testing market. Covid-19 antigen test developers could face a similar dilemma. Because antigen tests have lower specificity than PCR tests, antigen tests can generate higher levels of false positives in low-prevalence settings, that is when a person's probability of infection and a test's false positive rate are similar. Because of this, a confirmatory PCR test is still required if an asymptomatic patient tests positive, or if a symptomatic patient tests negative with an LFIA antigen test.

Use of PCR techniques in point of care situation still suffers from time consuming preparation steps of samples to be tested such as purification of the samples, etc. Thus, there is still a demand for compositions which prepare the biological samples such as saliva or nasopharygeal secrets in a manner that they can directly be used in a subsequent PCR-Test and which are suitable for POC.

It has surprisingly been found that a liquid composition with a low concentration of a chaotropic salt can meet the demands required for a Point-of-Care PCR analysis.

One embodiment of the present invention is a liquid composition for the preparation of a biological sample for methods assaying the presence of SARS-CoV-2 or genetic variations (mutant) of SARS-CoV-2 wildtype in said sample by POC PCR comprising:
a) a chaotropic salt, preferably guanidinium thiocyanate, in a concentration ranging from 1 to 960 mM and
b) optionally one or more RNAse inhibitor.

Another aspect of the present invention is a liquid composition for methods for assaying the presence of SARS-CoV-2 or genetic variations (mutant) of SARS-CoV-2 wildtype in said sample by POC PCR comprising a chaotropic salt, preferably guanidinium thiocyanate, in a concentration ranging from 1 to 960 mM.

Typical variants of SARS CoV-2 are listed in the following Table 1.

**Table 1:**

| **WHO label** | **Lineage + additional mutations** | **Country first detected (community)** | **Spike mutations of interest** |
|---|---|---|---|
| **Beta** | B.1.351 | South Africa | K417N, E484K, N501Y, D614G, A701V |
| **Gamma** | P.1 | Brazil | K417T, E484K, N501Y, D614G, H655Y |
| **Delta** | B.1.617.2 | India | L452R, T478K, D614G, P681R |
| **Omicron** | B.1.1.529 | South Africa and Botswana | (x) |

| | | | |
|---|---|---|---|
| x: A67V, Δ69-70, T95I, G142D, Δ143-145, N211I, Δ212, ins215EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981FAn essential component of the composition of the present invention is a chaotropic salt. Chaotropic salts are substances which disrupts the structure of, and denatures, macromolecules such as proteins and nucleic acids (e.g. DNA and RNA). Chaotropic solutes increase the entropy of the system by interfering with intermolecular interactions mediated by non-covalent forces such as hydrogen bonds, van der Waals forces, and hydrophobic effects. Macromolecular structure and function is dependent on the net effect of these forces (see protein folding), therefore it follows that an increase in chaotropic solutes in a biological system will denature macromolecules, reduce enzymatic activity and induce stress on a cell (i.e., a cell will have to synthesize stress protectants). Tertiary protein folding is dependent on hydrophobic forces from amino acids throughout the sequence of the protein. Chaotropic solutes decrease the net hydrophobic effect of hydrophobic regions because of a disordering of water molecules adjacent to the protein. This solubilises the hydrophobic region in the solution, thereby denaturing the protein. This is also directly applicable to the hydrophobic region in lipid bilayers; if a critical concentration of a chaotropic solute is reached (in the hydrophobic region of the bilayer) then membrane integrity will be compromised, and the cell will lyse. | | | |

Chaotropic salts that dissociate in solution exert chaotropic effects via different mechanisms. Salts can have chaotropic properties by shielding charges and preventing the stabilization of salt bridges. Hydrogen bonding is stronger in nonpolar media, so salts, which increase the chemical polarity of the solvent, can also destabilize hydrogen bonding. Mechanistically this is because there are insufficient water molecules to effectively solvate the ions. This can result in ion-dipole interactions between the salts and hydrogen bonding species which are more favorable than normal hydrogen bonds.

Common chaotropic salts are selected from the group consisting of guanidinium chloride, lithium perchlorate, lithium acetate, magnesium chloride, sodium dodecyl sulfate, and guanidinium thiocyanate.

Most effective results could be achieved with guanidinium salts such as guanidinium thiocyanate as the chaotropic salt.

For the purpose of the present invention and its use in point of care PCR analytics the concentration of the chaotropic salt should be adjusted to a range below 1 M. High concentrations of chaotropic salts leads to lysis of SARS CoV-2 and realease of viral RNA. However, a too low concentration leads to an inactivation of virus without lysis. Thus, the concentration of the chaotropic salt must be sufficient to perform lysis of the virus. On the other hand high concentrations of chaotropic salts inactivate nucleases, most importantly RNA degrading enzyme RNAse from host and pathogen. It has been found that low concentrations of chaotropic salts are sufficient to stabilize virla RNA and to inactivate RNAses.

Concentrations of 1 M or more of chaotropic salt, in particular guanidinium thiocyanate, are problematic since these concentrations are inhibiting polymerases such as reverse transcriptase and taq polymerase. Therefore high concentrations are useless for direct PCR without elimination of chaotropic salt. Dillution with liquid would also help but reduce viral concentration (losing sensitivity). Surprising is that submolar concentrations (mM) are not inhibiting PCR enzymes but sufficient to inactivate SARS-CoV-2 and inactivate RNAses.

The composition of the present invention preferably comprises the chaotropic salt, most preferably a guanidinium salt, especially guanidinium thiocyanate, in a concentration of 1 to 500 mM, preferably 2 to 250 mM, especially 5 to 100 mM or 10 to 90 mM, in particular 40 to 60 mM.

In a preferred aspect of the invention the composition of the invention is an aqueous composition.

For a point of care diagnostic biological samples are preferably easy accessible from patients and can be obtained even by non-medical trained people. In particular for the diagnostic of infections with SARS CoV-2 virus or mutants thereof biological samples are preferably selected from saliva and/or nasopharygeal secret.

Said biological samples can be extracted from patients by a swab or other extraction devices commonly known to the person skilled in the art.

In a further aspect of the present invention the composition preferably comprises two or more RNAse inhibitors, which are different form each other. Preferably the RNAse inhibitors are protein based RNAse inhibitors.

In a further aspect of the invention the composition comprises a recombinant RNAse inhibitor, preferably two or more recombinant RNAse inhibitors.

The recombinant RNAse inhibitor is a murine RNAse inhibitor which inhibits RNAse A, preferably inhibits RNases A, B and C.

Preferably the murine RNase inhibitor is a 50 kDa recombinant protein of murine origin. The inhibitor specifically inhibits RNases A, B and C. It inhibits RNases by binding noncovalently in a 1:1 ratio with high affinity. In one aspect it is not effective against RNase 1, RNase T1, S1 Nuclease, RNase H or RNase from Aspergillus. In addition, no inhibition of polymerase activity is observed when RNase Inhibitor is used with Taq DNA Polymerase, AMV or M-MuLV Reverse Transcriptases, or Phage RNA Polymerases (SP6, T7, or T3).

It has been surprisingly found that a composition which preferably comprises a murine RNAse inhibitor, which preferably inhibits RNAse A, B and C, shows an advantage effectivity.

In a preferred embodiment the composition comprises a monoclonal antibody to ribonuclease A.

Especially preferred are compositions comprising murine RNAse inhibitor and a monoclonal antibody to ribonuclease A.

Protein based RNAse inhibitors such as natural murine RNAse inhibitor as well as antibody based inhibition are known to inhibit RNAses but enhanced effect of combinatorial inactivation by antibody and murine RNAse inhibitor is surprising - indicating synergy and more effectivity in RNA stability. Synergy indicates different mode of action which is surprising because expert would expect inhibition of active center.

In a further preferred aspect the composition of the invention comprises monoclonal anti-spike Protein binding AND SARS Cov-2 neutralizing antibody which is preferably coated on a nanoparticle.

In a preferred embodiment the nanoparticle are iron particle which are preferably coated with silica. It has surprisingly found that the particles have an affinity to nucleic acid material and thus may enrich the SARS CoV-2-Virus material on the particles.

Without being bound by theory it is assumed that the nanoparticles do not sediment and float in the composition. Thus, the nanoparticles may be used to selectively purify the virus nucleic acid material.

The particles preferably have a volume average particle diameter of 1 to 150 nm, preferably 1 to 100 nm, especially 2 to 50 or 3 to 20 nm, determined by light scattering method.

The use of virus neutralizing antibodies, e.g. Covid-19 - human receptor binding inhibitor in therapy is known, however the use of a therapeutical antibody in biotech diagnostic applications shows surprising advantages. It not only leads to an inactivation and reduction of infectious risk but also leads to an immobilization of Covid-19 antibody on floating nanoparticles bind and enrich / increase local concentration of virus RNA.

In a further aspect of the invention the composition further preferably comprises nanoparticles which are coated with anti-Spike SARS-Cov-2 antibodies and/or biotin and/or protein A.

A further embodiment of the present invention is a POC PCR method for the detection of SARS-Cov-2 or mutants thereof comprising the steps:
a) Mixing a biological sample with a composition of the invention to obtain a mixture A,
b) optionally incubating mixture A,
c) mixing mixture A with a PCR reaction mixture preferably comprising one or more of reverse transcriptase, polymerase, PCR buffer, primer and probes for a PCR assay.

In a preferred aspect of the present method step a) is carried out in a container which is adapted to be sealed with a cap wherein said cap preferably has a through hole with a closure element which is separably attached.

Preferably step b) is carried out in a container, preferably a tube, comprising mixture A sealed with a cap wherein said cap preferably has a through hole with a closure element which is separably attached.

This has the advantage that mixture A can be released from the container through the through hole in a drop-wise form. Further the risk for a contamination of the operator is minimized.

Step c) is preferably carried out by releasing the mixture A from said container through the through hole into said PCR reaction mixture.

The PCR reaction mixture comprises primer and probes which are suitable for the detection of SARS-Cov-2 or mutants thereof. Suitable PCR reaction mixtures and especially primer and probes are known to the person skilled in the art.

A further embodiment of the present invention is the use of the composition the present invention for a POC PCR assay.

A further embodiment of the present invention is a kit comprising a container containing the composition of the present invention and a separate container comprising a PCR reaction mixture preferably comprising one or more of reverse transcriptase, polymerase, PCR buffer, primer and probes for a PCR assay.

In a preferred embodiment the container comprising the composition according to the invention is a container which is sealed with a cap wherein said cap has a through hole with a closure element which is separably attached.

The present invention provides a fast and effective diagnostic method for the detection of SARS CoV-2 virus or mutants thereof at a point of care (POC). Standard RNA extraction methods and RT qPCR reactions need 60-90 minutes even with automation. The present invention reduces the RNA isolation to a single step which may need 5 minutes of incubation. The samples can directly used for the PCR analysis, which preferably is a multiplex RT qPCR. The time from sample extraction from the patient to the result may be within 35 minutes.

### Example:

### Composition 1:

An exemplary composition of the invention contains an aqueous formulation comprisiong:
50 mM Guanidium Thiocyanate
Detergent
Covid-19 neutralizing antibody
RNAse Inhibitor 1: murine RNAseA inhibitor
RNAse Inhibitor 2: anti RNAseA antibody
iron nanoparticles which are coated with silica and wherein said coated nanoparticles are coated with protein A and biotin and said Covid-19 neutralizing antibody

### Composition 2:

An exemplary PCR reaction mixture for a 1-Step RT qPCR containing:
MluV reverse transcriptase
Taq Polymerase
PCR buffer
primer and probes suitable for the detection of SARS-Cov-2 or mutants thereof and for Taqman assay

### Exemplary procedure

A nasopharyngeal sample material is taken with a swab and the tip of the swab is swirled in the provided tube with composition 1 4-5 times. The tube is sealed with a cap wherein said cap has a through hole with a closure element which is separably attached.

The obtained mixture is incubated at 25 °C for 5 minutes.

Subsequently the closure element is separated to open the through hole in the cap.

The incubated mixture is used directly for 1 step RT qPCR reaction - 1 drop corresponding to 25-30 µl of volume is transferred through the through hole into a PCR tube containing the RT qPCR reaction mixture (Composition 2).

### Ring study samples

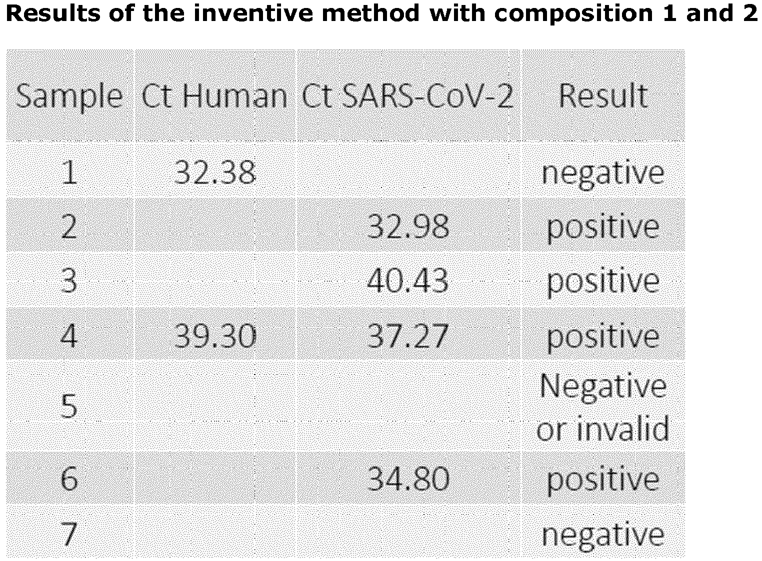

### Results

### Inventive method versus Regular PCR versus Abbot POC

Tested at: DiagnosticLab Tauernklinikum Zell am See

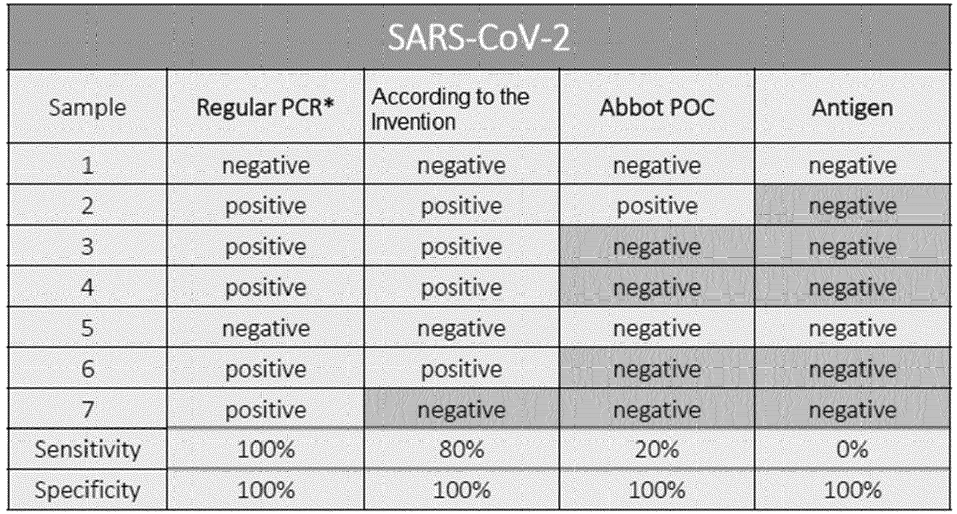

### Conclusion

The method of the invention with composition 1 and 2 in a POC PCR demonstrate a clinical sensitivity of >90 % and a specificity of 100 %. A commercially available POC PCR system of Abbott shows a sensitivity ~ 20% and a specificity of 100 %. Anti-genetic tests could not demonstrate a single positive sample.

## Claims

1. Liquid composition for the preparation of a biological sample for methods for assaying for the presence of SARS-CoV-2 or genetic variations (mutant) of SARS-CoV-2 wildtype in said sample by POC PCR comprising:
a) a chaotropic salt, preferably guanidinium thiocyanate, in a concentration ranging from 1 to 960 mM and
b) optionally one or more RNAse inhibitor.

2. Composition according to claim 1 wherein the biological sample is saliva and/or nasopharygeal secret.

3. Composition according to claim 1or 2 wherein guanidinium thiocyanate is present in a concentration of 1 to 500 mM, preferably 2 to 250 mM, more preferably 5 to 100 mM or 10 to 90 mM, especially 40 bis 60 mM

4. Composition according to one or more of the preceding claims comprising two or more RNAse inhibitors.

5. Composition according to one or more of the preceding claims comprising two or more recombinant RNAse inhibitors.

6. Composition according to one or more of the preceding claims comprising a murine RNAse inhibitor, which preferably inhibits RNAse A, B and C.

7. Composition according to one or more of the preceding claims comprising a monoclonal antibody to ribonuclease A.

8. Composition according to one or more of the preceding claims comprising monoclonal anti-spike Protein binding AND SARS Cov-2 neutralizing antibody.

9. Composition according to one or more of the preceding claims comprising nanoparticles which are coated with anti-Spike SARS-Cov-2 antibodies and/or biotin and/or protein A.

10. POC PCR method for the detection of SARS-Cov-2 or mutants thereof comprising the steps:
a) Mixing a biological sample with a composition according to one or more of claims 1 to 9 to obtain a mixture A,
b) optionally incubating mixture A,
c) mixing mixture A with a PCR reaction mixture preferably comprising one or more of reverse transcriptase, polymerase, PCR buffer, primer and probes for a PCR assay.

11. Method according to claim 10 wherein step a) is carried out in a container which is adapted to be sealed with a cap wherein said cap preferably has a through hole with a closure element which is separably attached.

12. Use of a composition according to one or more of claims 1 to 9 for a POC PCR assay.

13. Kit comprising a container containing the composition according to one or more of claims 1 to 9 and a separate container comprising a PCR reaction mixture preferably comprising one or more of reverse transcriptase, polymerase, PCR buffer, primer and probes for a PCR assay.

14. Kit according to claim 13 wherein the container comprising the composition according to one or more of claims 1 to 9 is a container which is sealed with a cap wherein said cap has a through hole with a closure element which is separably attached.
